# EUROPEAN PATENT SPECIFICATION

(11) **EP 1 863 443 B1**
(45) Date of publication and mention of the grant of the patent: **21.10.2009**
(21) Application number: 06711375.3
(22) Date of filing: 02.03.2006
(51) Int. Cl.: A61K 9/00

(54) **RAPIDLY DISINTEGRATING DOSAGE FORM COMPRISING MAGNESIUM CARBONATE HEAVY**
SCHWERES MAGNESIUMCARBONAT ENTHALTENDE SCHNELL AUFLÖSENDE DOSIERFORM
FORME POSOLOGIQUE A DESINTEGRATION RAPIDE COMPRENANT DU CARBONATE DE MAGNESIUM LOURD

(30) Priority: 02.03.2005 IS 7724
(43) Date of publication of application: 12.12.2007
(73) Proprietor: Actavis Group hf., 220 Hafnarfjordur (IS)
(72) Inventor: KRISTJANSSON, Torfi E., IS-108 Reykjavik (IS)
(74) Representative: Arnason Faktor
(86) International application number: PCT/IS2006/000005
(87) International publication number: WO 2006/092812

(56) References cited:
- WO-A-20/04091585
- WO-A-20/05065645
- GB-A- 1 422 176
- US-A1- 2002 137 771
- US-B1- 6 635 278
- ANONYMOUS: "Granted Marketing Authorisations: PL 00010/0350" MHRA, MEDICINES AND HELTHCARE PRODUCTS REGULATORY AGENCY, [Online] October 2005 (2005-10), - November 2005 (2005-11) XP002397595 Dep. of Health of British Governement, London Retrieved from the Internet: URL:http://www.mhra.gov.uk/home/idcplg?Idc Service=SS_GET_PAGE&nodeId=124>

## Description

### FIELD OF THE INVENTION

The present invention relates to compositions for solid pharmaceutical dosage forms, which disperses rapidly when in contact with moisture.

### BACKGROUND OF THE INVENTION

Dosage forms which disintegrate rapidly prior to swallowing, provide convenient means to administer pharmaceuticals to patients in the need thereof, e.g. to those who have difficulties in swallowing and also when the active compound needs to be administered in large doses to provide a desired therapeutical effect, In particular when rendered difficult to produce readily swallowable tablets of convenient size.

Two dosage forms, dispersible tablets and orodispersible tablets, address the above problem. The dispersible tablets disintegrate in water prior to administration while the orodispersible tablets disintegrate when contacted by the moisture in the lining of the mouth.
Both these forms have clear advantages over the traditional tablets which need to be swallowed. By dissolving the tablets first in water, large amount of medication can be administrated to patients who might otherwise have difficulties swallowing the same amount of medicament in a tablet form. Clearly, it is of advantage when the dispersible tablets disintegrate quickly in the water medium prior to swallowing.
Likewise, the advantages of orodispersible tablets are obvious where the administration has to occur in the absence of available water or when the release of the medication is rapidly needed. Furthermore, patients like the very young or elderly, the non-complying and those with physical impairment are all likely to be in the need of orodispersible tablets when being administrated medication, in such cases administration of a full glass of liquid may be unsuitable. Orodispersible tablets, which are also known as smelt tablets or rapid oral release tablets, need to disintegrate quickly, such as at least in less than 3 minutes, but preferably they should disintegrate within seconds of contacting the lining of the buccal cavity.

The unique nature of both of these tablet-forms, which are generally meant to be non-chewable and to rapidly disintegrate once in contact with moisture, inherently brings several challenges which must be circumvented during manufacturing. These tablets must preferably be compressed with low pressure, to ensure that they are porous, which augments rapid dissolving, once in contact with the moisture. Concomitantly the tablets must have low friability and be able to meet the manufacturing methods most commonly used in the pharmaceutical industry. Preferably, the composition should be sufficiently versatile, to allow the compounding to be made either with a process using granulation or direct compression. The composition should preferably be made up of Insoluble ingredients, as soluble components tend to absorb water and therefore the tablet becomes densely hydrated and slowly dissolves instead of rapidly dispersing the tablet. These tablets should also preferably be bioequivalent to tablets of identical dosages which are made for conventional release, i.e. in the stomach or the intestine.

A skilled person in the art is well aware that there are several compounding methods for manufacturing dispersible or orodispersible tablets. For compounding orodispersible tablets, several approaches have traditionally been used. For example US patent No. 6,083,531 describes the formation of rapid oral dispersing tablets, where the components are mixed together, and dispensed on the final packaging material before being dried and shaped. This is similar to the well known Zydis® system, which uses freeze-drying of the dispensed components once placed on the packaging material. These tablets disintegrate very rapidly, due to absence of moisture and the porous nature of the tablets, which promotes a rapid ingression of water. However, both methods are limited by the fact that special processing units are needed for the manufacturing of the tablets, which are not commonly available in pharmaceutical companies in addition to be commercially expensive.
Conversely, a technology for generating orodispersible tablets is often based on dosage forms which disintegrates due to the porous nature of the tablets, which commonly includes soluble excipients, for example mannitol or sorbitol, in addition to an effervescent agent like sodium carbonate and a weak acid, typically citric acid, which further enhances the disintegration of the tablets in the buccal cavity. For example, US patent No. 6,328,994 describes orally disintegrating tablets formed by coating granules with lansoprazole, which are again coated with an enteric coating layer, the granules are subsequently mixed with a disintegrant and compressed into tablets. The granules may contain a basic inorganic salt, e.g. magnesium carbonate to stabilize the active ingredient, which is then acting as buffer.
In the patent EP 636,364 an orally disintegrating tablet is described, wherein the disintegration is brought about by a water-disintegratable, compressible carbohydrate and a binder.

US patent No. 5,178,878, describes a composition with an effervescent agent which aids the disintegration of the tablet once in the buccal cavity.

In US patent No. 5,464,632, a mixture of excipients imparts the disintegration of multiparticulate tablets In the mouth in less than 60 seconds.

Effervescent ingredients are also commonly used for dissolving dispersible tablets in water which are then subsequently drunk as a means to administrating the medicament. Effervescent tablets are however not generally suitable for administration as orodispersible tablets, as the effervescence effect (generation of carbon dioxide) by an effervescent tablet in the mouth Is often considered unpleasant.

US patent No. 6,635,278 discloses use of magnesium carbonate heavy in tablets with the active ingredient adefovir dipivoxil. The exemplified tablet formulations are conventional hard tablets intended to be swallowed to dissolve in the stomach.

GB-A-1422176 likewise discloses tablets containing magnesium carbonate heavy which are conventional hard tablets for dissolution in stomach, that dissolve slowly in water.

Magnesium carbonate heavy is used as excipient in "Rennie Deflatine" tablets with simeticone active ingredient; these are chewable tablets.

WO 2004/091585 discloses orally disintegrating tablets comprising silicified microcrystalline cellulose, e.g. ProSolv 50™ or ProSolv 90™.

US patent No. 6,521,256 discloses a benzimidazole anti-ulcer medicament comprising a basic inorganic salt like magnesium carbonate heavy as stabilisation agent.

Unfortunately, none of these above inventions provides a method which is versatile i.e. can be used both for compounding tablets using wet granulation and direct compression processes and the composition can be used either for orodispersible tablets or dispersible tablets.

### SUMMARY OF THE INVENTION

The technical problem underlying the present invention is to find a method to generate suitable dosage forms which have concomitantly all the essential features of orodispersible and dispersible tablets and can be adjusted to different tabletting techniques in addition to meeting all the standards currently required for solid dosage forms containing a medicament, such as hardness and friability.
The solution to the above technical problem is achieved by providing the embodiments of the present invention which are characterized in the claims and described in further detail herein.

Surprisingly and by counterintuitive insight, it was discovered that magnesium carbonate heavy, Is a key ingredient for solving the above technical hurdles, and allowing the compounding of a rapid disintegrating dosage form with the aforementioned required characteristics. Magnesium carbonate has been described in two different forms generally referred to as 'heavy' and 'light'. Generally, magnesium carbonate heavy may be regarded as a tetrahydrate with the formula [(MgCO₃)₃·Mg(OH)₂·4H₂O] while magnesium carbonate light may be considered as [(MgCO₃)₃·Mg(OH)₂·3H₂O] or trihydrate form. Magnesium carbonate is insoluble in water, 95% ethanol and other solvents. However, it dissolves and effervesces In dilute acid solutions and Is also soluble in water containing carbon dioxide. Magnesium carbonate has good dispersing properties once in contact with the gastric acid in the stomach and quickly dissolves, forming magnesium chloride and carbon dioxide. However, in the present invention it was surprisingly discovered that while still in an Insoluble form, magnesium carbonate heavy facilitates rapid dispersion of the compressed dosage form upon contact with neutral water or in a basic environment, e.g. provided by in the mouth.

Furthermore, the present invention provides methods which are versatile for compounding orodlspersible tablets which rapidly disintegrate in the buccal cavity, and have a pleasant mouth-feel. The invented formulation was also found to be bio-equivalent when tested against dosage forms of equal strength which disintegrate in the stomach or the intestines. The current teaching also discloses a method for compounding a dispersible dosage form which rapidly disperses in water prior to administrating the medicament and is also bio-avallable. The tablets of the present invention, although not limited to, generally have a weight in the range from about 65 to about 520 mg and can accommodate the active pharmaceutical ingredient when either in uncoated or in coated particles. The composition of the tablet can either be with or without water-soluble excipients. By using magnesium carbonate heavy it has been surprisingly found that one can simultaneously obtain an organoleptically pleasing dosage form, which concomitantly also results in a dispersible or a orodispersible dosage form and which has a pleasant mouth-feel.

Furthermore, this invented composition can accommodate a wide variety of active ingredients and as well a wide selection of excipients, such as diluents, binders, lubricants, glidants, taste maskings and coatings.

### DETAILED DESCRIPTION OF THE INVENTION

The present invention Is directed to a rapid disintegrating solid dosage form. These dosage forms disintegrate rapidly in water, and can be suitably dispersed in water prior to administration. Dosage forms intended to be dispersed in water prior to administration are generally referred to as dispersible tablets. In preferred embodiments, the dosage forms are formulated such that they can disintegrate comfortably in the mouth, without prior dispersion in water. Such tablets which are suitable and intended for such administration are referred to as orodispersible tablets. It follows that orodispersible dosage forms are generally also 'dispersible', i.e. can generally be dispersed in water prior to administration, for example if the patient prefers such mode of administration. Dispersible dosage forms, however, are not all necessarily orodispersible, or at least not necessarily suitable as such, for example due to negative organoleptic characteristics, unpleasant mouth-feel or the like.

The tablets of the dosage forms preferably have concomitantly a friability which is less than 1%, when tested according to the outlines provided by the European Pharmacopoeia. Despite the fact that these tablets have low friability and disintegrate rapidly, the hardness of the tablets can range from 15 to 170 N, such as in the range of 15-100 N for the largest dosage forms.

To accommodate a wide variety of pharmaceutical ingredients, compounding techniques cannot solely rely on a one single method. The dosage forms and compositions disclosed herein are useful for compounding tablets, using either direct compression or wet granulation, for generating either orodispersible tablets or dispersible tablets.

Not only does the inventive composition disclosed herein allow the tablets to disintegrate rapidly, once in contact with the moisture, but also preferably results in tablets which have pleasant mouth-feel and positive organoleptic sensation.

It is within the scope of the present invention to accommodate ingredients which need to be coated, e.g. due to unpleasant taste or unstable components, or for any other reason, provided that the coating does not interfere with the disintegration of the dosage form.

The current invention uses magnesium carbonate heavy, for rapidly dispersing the dosage forms once in contact with moisture. The tablets can contain an amount in the range between 1 to 85 % by weight of magnesium carbonate heavy, more preferably in the range between 4 and 75 wt%, such as in the range of 4 and 65% by weight of magnesium carbonate heavy for rapid orodispersible or for dispersible dosage forms, e.g. In the range of 20-75 wt%, such as

in the range of 20-65% by weight, such as in the range of about 30-65 wt%, or in the range of about 20-50%, including the range of about 25-45% by weight. In some embodiments a small amount of magnesium carbonate heavy is found to be sufficient, such as in the range of 4-12% by weight, including the range of 4-8% by weight, such as, e.g. about 5%, 6%, 7% or 8% by weight.

The Incorporation of magnesium carbonate heavy in the dispersible dosage form, as provided by the invention, facilitates the disintegration of the tablet in water within three minutes, preferably within two minutes, or more preferably within one minute, or even faster such as within 30 seconds.

Orodispersible dosage forms generally need to disperse even more quickly than dispersible dosage forms, and the orodispersible dosage forms of the present invention, as described herein, disintegrate in the buccal cavity within two minutes and more preferably within one minute and even more preferably within 30 seconds or shorter time, such as within 20 seconds or more preferably within 15 seconds and even more preferably within 10 seconds. Such a rapid dispersion is achieved by incorporating magnesium carbonate heavy into tablets as described herein.

The use of magnesium carbonate heavy was surprisingly found to result in pleasant mouth-feel and to work as an efficient dispersing agent without any effervescent effects under basic and neutral conditions.
The other excipients which can be used in accordance with the Invention can for example be chosen from, but are not limited to, commonly used diluents, binders, lubricants, taste masking agents, and coatings.
Diluents are for example, but not limited to: lactose, spray dried lactose, xylitol, sorbitol, calcium sulfate dihydrate, inositol, starch, sucrose, dextrose, dibasic calcium phosphate dihydrate, mannitol and microcrystalline cellulose. In particular, granulated spray-dried mannitol, such as e.g. Pearlitol SD200® is found to be useful, especially in formulations intended for direct compression of tablet dosage forms according to the invention.
Useful binders are for example but not limited to: sucrose, glucose, cellulose derivatives, polyvinyl pyrrolidone (PVP), hydroxymethylcellulose, ethylcellulose, tragacanth, gelatin, sodium alginate, polymetacrylates, pregelatinized starch and hydroxypropylcellulose.
Useful lubricants are for example, but not limited to, sodium stearate, waxes, calcium stearate, stearic acid, talc, magnesium stearate, hydrogenated vegetable oil, boric acid, sodium chlorate, carbowax 4000 and 6000, sodium oleate, sodium acetate, magnesium lauryl sulfate, sodium benzoate, DL-leucine, sodium benzoate and sodium lauryl sulfate.
In some embodiments, low substituted hydroxypropyl cellulose is particularly useful, which can be In an amount in the range of about 2-10 wt%, such as in the range of about 3-6 wt%, e.g .about 3 wt%, about 4 wt%, about 5 wt% or about 6 wt%.

The formulation also allows for the incorporation of glidants, for example but not limited to: talc or cornstarch.

Particular preferred compositions for the dosage forms of the invention comprise in addition to the magnesium carbonate heavy Avicel® CE-15 (FMC BioPolymer, Philadelphia, USA) which is a specially engineered particle component comprising microcrystalline cellulose and guar gum, or other chemically equivalent components of other brands. Avicel® CE-15 is intended in particular for chewable dosage forms and has to our knowledge not been suggested for dispersible dosage forms. The dosage form tablets of the present invention preferably comprise in the range of 3-15 wt% Avicel CE-15, and more preferably In the range of about 3-10 wt%, e.g .about 10 wt%, or in the range of about 3-8 wt%, such as about 4 wt%, about 5 wt%, about 6 wt%, about 7 wt% or about 8 wt%.

As can be seen in the accompanying examples, it is found beneficial to use fine particle size microcrystalline cellulose as an excipient, both for direct compression and wet compression of the dosage forms of the invention. As an example , Avicel® PH-102 (FMC BioPolymer, Philadelphia, USA) can be used, but also other similar cellulose types with similar material characteristics, e.g. silicified microcrystalline cellulose (e.g. Prosolv SMCC-90 (JRS Pharma, PAtterson, NY, USA)), "UICEL" and the like. "UICEL" is a sodium hydroxide treated cellulose powder (see Kumar, V. et al. Int J Pharm. 2002 Mar 20;235(1-2):129-40).
Such fine microcrystalline cellulose is preferably comprised in the tablets of the invention in and amount in the range of about 5 to about 40 wt%, and preferably in the range of about 8-30 wt%, such as in the range of about 10-20 wt%, such as about 8 wt%, about 10 wt%, about 15 wt%, abpit 20 wt%, about 25 wt% or about 30 wt%.
In addition to using magnesium carbonate heavy, the composition can also include further disintegrants, for example, but not limited to: starch, pre-gelatinized starch and other modified starch, crosslinked sodium carboxymethyl cellulose, magnesium aluminum, silicate, sodium starch glycollate, cellulose, gums and crosslinked polyvinyl pyrrolidone (crospovidone) is particularly preferred. Such additional disintegrant, if found beneficial, may be comprised in an amount in the range of about 5-20 wt%, or more preferably in the range of about 5-1 5 wt%, such as In the range of about 8-12 wt%, e.g. about 8 wt%, 10 wt%, or about 15 wt%.

The formulation of the present invention may include other conventional ingredients known in the art for improving the dosage form. These could for example include, but are not limited to, natural and artificial flavoring agents, such as mannitol, sorbitol, maltitol, xylitol, N-α-L-Aspartyl-L-phenylalanine 1-methyl ester (aspartame) and 6-methyl-3,4-dihydro-1,2,3-oxathiazin-4(3H)-one-2,2-dioxide, particularly the potassium salt thereof (acesulfame K), natural and synthetic flavors, such as tartaric acid. Those skilled in the art of compounding pharmaceutical medicaments will appreciate that the amount of flavoring and sweeteners, if any, present in the composition will be directly proportional to the bitterness which has to be masked.

The present invention can accommodate a wide variety of pharmaceutical active Ingredients. As illustrated by the Examples herein, the formulations and methods of the invention have been demonstrated to provide dispersible or orodispersible dosage forms with chemically different active substances, indicating that the present invention applies generally to different compounds that encompass different chemical characteristics, e.g. with respect to stability, hydrophobicity, material properties or crystal forms.

Examples of pharmaceutically active ingredients that can be formulated into either the orodispersible or the dispersible dosage form, either alone or in combination include antihistamines, anti-motion sickness agents, analgesics, and anti-inflammatory agents (e.g., indomethacin, aspirin, ibuprofen, naproxen, ketorolac, COX-1 inhibitors, COX-2 inhibitors, and the like), antibiotics, cholesterol lowering agents, anti-anxiety agents, anti-hypertensives, antiangiogenic agents (e.g., combrestatin, contortrostatin, anti-VEGF, and the like), anticancer agents (e.g., mechloretharine, cyclophosphamide, fluorouracil, thioguanine, carmustine, lomustine, melphalan, chlorambucil, streptozocin, methotrexate, vincristine, bleomycin, vinblastine, vindesine, dactinomycin, daunorubicin, doxorubicin, tamoxifen, and the like), hypnotics, narcotics (e.g., morphine, meperidine, codeine, and the like), local anesthetics (e.g., the amide- or anilide-type local anesthetics such as bupivacaine, dibucaine, mepivacaine, procaine, lidocaine, tetracaine, and the like), anti-ulcer agents, coronary dilators, antivirals, anti-psychotics, anti-depressants, neuromuscular agents, anti-diarrheals, hypoglycemic agents, thyroid suppressors, anabolic agents, antispasmodics, antimigraine agents, diuretics, stimulants, decongestants, uterine relaxants, anti-arrhythmics, male erectile dysfunction compounds including slidenafil and tadalafil, Maxi-K channel openers or neuroprotective agents for the treatment of stroke or Alzheimer's disease and therapeutically appropriate combinations thereof.

Examples of specific therapeutic agents include serotonin 5-HT reuptake inhibitors such as escitalopram, citalopram, fluoxetine, fluvoxamine, paroxetine, sertraline and venlafaxine, different heterocyclic compounds both non-polar, slightly polar, including thiobenzodiazepines such as olanzapine and clozapine; benzimidazole derivatives such as lansoprazol, timoprazol, esomeprazole, omeprazol, pantoprazol, leminoprazol, pariprazol; also bupropion, nefazodone; phenyltriazine derivatives including lamotrigine, dihydropyridine calcium channel blockers, . e.g. nifedipine, nimodipine, nisoidipine, nicardipine, amiodipine, etc.; statins e.g. atorvastatin, fluvostatin, simvastatin, lovastatin, etc., anticonvulsants, e.g. phenytoin, carbamezepine, etc.; analgesics, steroids, e.g. prednisone, prednisolone, hydrocortisone, etc.; fibrates, e.g. gemfibrozil, fenofibrate, clofibrate, etc., mirtazapine, and benzisoxazole-derivatives such as risperidone.

The invention described herein, is suitable for generating tablets by either using wet granulation or direct compression. During the wet granulation process, the ingredients are blended, moistened with a granulation liquid and agglomerates are built up. The wetting process is continued until a desired homogeneity is achieved, with subsequent drying of the wet mass. The mixture is subsequently sieved, optionally mixed with additional ingredients, and compressed into tablets. Conversely, for direct compression, various components are mixed together, until homogeneity is achieved, sieved and subsequently compressed into a dosage form. The direct compression is generally superior to most other tabletting methods, as it is more economical. The active ingredient and/or the tablets themselves can be coated or uncoated.

Dispersible tablets in accordance with the present are intended to be dispersed in water prior to administration, resulting in a homogenous dispersion. However, the dispersible tablets can generally also be chewed and/or swallowed.

Orodispersible dosage form, In the context of the present invention is understood to mean a tablet which starts to disintegrate when in contact with the moisture of the lining of the buccal cavity before being swallowed. However, the orodispersible tablets can also be dispersed in water prior to administration and subsequently drunk or they can be chewed and/or swallowed.

The use of magnesium carbonate heavy for dispersing medicaments under neutral or basic conditions, prior to ingestion, or within the digestive system, or subcutaneously, or on the surface of the body, or in any other means or forms, for the purpose of augmenting the delivery of the active ingredient, is also understood to be included In the present invention.

The disintegration test determines whether tablets disintegrate within the prescribed time when placed in a liquid medium. Disintegration Is considered to be achieved when: a) no residue remains on the screen, or b) if there is a residue, it consists of a soft mass having no palpably firm, unmoistened core, or c) only fragments of the coating remains on the screen.

To determine if the surfaces of the tablets are damaged and/or showing any evidence of lamination or breakage when subjected to mechanical shock or attrition a test of friability is carried out, essentially as is described by the European Pharmacopoeia.

It is to be understood that various other embodiments and modifications of the present invention are apparent to a person skilled in the art, without departing from the scope and the spirit of the invention as described. Accordingly, it is not intended that the scope of the claims appended hereto and following examples to be interpreted to the exact description set forth, but rather that the claims be construed as encompassing all of the features of patentable novelty that reside in the present invention, including all the features and embodiments that would be treated as equivalent thereof by those skilled in the art to which the invention pertains. The invention is further described with reference to the following experimental work.

### EXAMPLES

### Example 1

### Comparison of the time for disintegrating dispersible tablets containing lamotrigine, when compounded either with magnesium carbonate heavy or mannitol using wet granulation

Particle size for lamotrigine is 99.2% less than 120 µm and 5% less than 10 µm.

**Table 1: Tablets containing magnesium carbonate heavy.**

| | Ingredient | mg per tablet |
|---|---|---|
| 1 | Lamotrigine | 5 |
| 2 | Magnesium Carbonate Heavy | 122 |
| 3 | Microcrystalline Cellulose (Avicel PH-102) | 29 |
| 4 | Povidone | 4 |
| 5 | Hydroxypropyl cellulose low substituted (L-HPC, LH-11) | 8 |
| 6 | Saccharine sodium | 2 |
| 7 | Crospovldone (Polyplasdone XL-10) | 8 |
| 8 | Purified water | quantum satis |
| 9 | Crospovidone (Polyplasdone XL=10) | 8 |
| 10 | Microcrystalline cellulose + Guar Gum (Avicel CE-15) | 10 |
| 11 | Black currant flavour Silarom | 2 |
| 12 | Magnesium stearate non bovine | 2 |
| | | 200 |

The results for the 5 mg tablets were as follows:
Hardness: 39.2 N (35-49 N)
Disintegration: 20 sec (15°C water)
Friability: 0.06%

**Table 2: Tablets containing mannitol**

| | Ingredient | mg per tablet |
|---|---|---|
| 1 | Lamotrigine | 5 |
| 2 | Mannitol (Pearlitol SD 200) | 122 |
| 3 | Microcrystalline Cellulose (Avicel PH-102) | 29 |
| 4 | Povidone | 4 |
| 5 | Hydroxypropyl cellulose low substituted (L-HPC, LH-11) | 8 |
| 6 | Saccharine sodium | 2 |
| 7 | Crospovidone (Polyplasdone XL-10) | 8 |
| 8 | Purified water | quantum satis |
| 9 | Crospovidone (Polyplasdone XL-10) | 8 |
| 10 | Microcrystalline cellulose + Guar Gum (Avicel CE-15) | 10 |
| 11 | Black currant flavour Silarom | 2 |
| 12 | Magnesium stearate non-bovine | 2 |
| | | 200 |

The results were as follows:
Hardness: 43.5 N (40-49 N)
Disintegration: 40 sec (15°C water)
Friability: 0.16%

Ingredients 1-7; Avicel PH-102, povidone, Polyplasdone XL-10, lamotrigine, L-HPC, saccharine sodium and magnesium carbonate heavy or mannitol are added in this order to a bowl; mixed, sieved and re-mixed. The blend is wetted with ingredient 8, purified water, mixed further and then sieved. The granules are dried and sieved. Ingredients 9-11, Black currant flavour, Polyplasdone XL-10 and Avicel CE-15 are then added to the blend in this order and mixed. Finally ingredient 12, magnesium stearate, is added to the blend and mixed. The blend is then compressed into tablets.

The above comparison of the time for the disintegration of tablets, compounded either with magnesium carbonate heavy or mannitol, but otherwise identical in composition, clearly shows that a substantially shorter time of disintegration is obtained when using the former. This is due to the fact, that magnesium carbonate heavy which appears to form a porous structure or matrix within the tablets, is disrupted by the water. An important element of magnesium carbonate heavy is the fact that it is insoluble under basic and neutral condition. Mannitol, in contrast, solubilizes by absorbing available moisture, thereby limiting accessible water which otherwise facilitates disintegration of the tablet.

### Example 2

### Bio-study of the Lamotrigine dispersible tablets

The bio-study was a comparative, randomized, single-dose, 2-way crossover bio-availability study of: (A) Actavis Lamotrigine 5 mg dispersible tablets (according to Example 1a), (B) Lamictal® (Glaxo Smithkline) 5 mg dispersible tablets in 24 healthy subjects under fasting conditions. The results were as follows:

**Table 3: Pharmacokinetic parameters**

| | AUCₒ₋ₜ³ | AUC_{0-inf} | Cₘₐₓ |
|---|---|---|---|
| Ratio¹ | 103.79% | 103.69% | 103.57% |
| 90% Geometric C.I.² | 100.98% to 106.69% | 100.53% to 106.95% | 97.39% to 110.14% |
| Intra-Subject CV | 5.55% | 6.25% | 12.46% |

| | | | |
|---|---|---|---|
| ¹Calculated using least-squares means according to the formula: e^{(Lamotrigine(A)- Lamictal (B)} X100 ²90% Geometric Confidence Interval using In-transformed data ³t - 120 hours | | | |

Based on the bio-study, it is concluded that the two tablet types are bio-equivalent under fasting condition.

Examples 3 to 9 describe different compounding methods for dosage forms containing mirtazapine and a bio-availability study.
The particle size for mirtazapine is 90% less than 60 µm and 50% less than 30 µm.

### Example 3

### Compounding of orodispersible tablets containing mirtazapine using wet granulation

**Table 4: Composition**

| | Ingredient | mg per tablet |
|---|---|---|
| 1 | Mirtazapine | 45 |
| 2 | Magnesium Carbonate Heavy | 181.6 |
| 3 | Saccharine sodium | 4 |
| 4 | Microcrystaline cellulose silicified (Prosolv SMCC-90) | 43.5 |
| 5 | Mannitol | 65.25 |
| 6 | Povidone | 9 |
| 7 | Crospovidone (Polyplasdone XL-10) | 26.1 |
| 8 | Purified water | quantum satis |
| 9 | Microcrystaline cellulose silicified (Prosolv SMCC-90) | 21.75 |
| 10 | Blackcurrant flavour Silarom | 8.7 |
| 11 | Crospovidone (Polyplasdone XL-10) | 26.1 |
| 12 | Magnesium stearate non bovine | 4 |
| | | 435 mg |

The ingredients 1-7, mirtazapine, magnesium carbonate heavy, saccharine sodium, Prosolv SMCC-90, mannitol, povidone and Polyplasdone XL-10 are mixed, wetted with purified water, granulated, sieved and dried. Then ingredients 9-12, Prosolv SMCC-90, Blackcurrant flavour Silarom, Polyplasdone XL-10 and magnesium stearate are added to the blend, mixed and the blend is ready for compression.

The results were as follows:
Disintegration: 20 sec (37°C water)
Hardness: 30 - 42 N

### Example 4

### Compounding of orodispersible tablets containing mirtazapine using wet granulation

**Table 5**

| | Ingredient | mg per tablet |
|---|---|---|
| 1 | Mirtazapine | 15 |
| 2 | Magnesium Carbonate Heavy | 67.875 |
| 3 | Aspartame | 6 |
| 4 | Microcrystaline Cellulose (Avicel PH 102) | 15 |
| 5 | Hydroxypropyl cellulose low substituted (L-HPC, LH-11) | 6 |
| 6 | Crospovidone (Polyplasdone XL-10) | 9 |
| 7 | Purified water | quantum satis |
| 8 | Microcrystalline cellulose + Guar Gum (Avicel CE-15) | 15 |
| 9 | Orange flavour Silesia | 6 |
| 10 | Crospovidone (Polyplasdone XL-10) | 9 |
| 11 | Magnesium stearate non bovine | 1.125 |
| | | 150 |

The ingredients 1-6, mirtazapine, magnesium carbonate heavy, aspartame, Avicel PH 102, L-HPC and Polyplasdone XL-10 are mixed, wetted with purified water, granulated, sieved and dried. Then ingredients 8-11, Avicel CE-15, Orange flavour Silesia, Polyplasdone XL-10 and magnesium stearate are added to the blend, mixed and the blend is ready for compression.

The results were as follows:
Disintegration: 15 sec (37°C water)
Hardness: 15 - 23 N
Friability: 0.67%

### Example 5

### Compounding of orodispersible tablets containing mirtazapine using direct compression

**Table 6: Composition**

| | Ingredient | mg per tablet |
|---|---|---|
| 1 | Mirtazapine | 15 |
| 2 | Mannitol DC (Pearlitol SD 200) | 60.75 |
| 3 | Magnesium Carbonate Heavy | 7.5 |
| 4 | Microcrystalline Cellulose (Avicel PH-102) | 30.375 |
| 5 | Hydroxypropyl cellulose low substituted (L-HPC, LH-11) | 6 |
| 6 | Crospovidone (Polyplasdone XL-10) | 12 |
| 7 | Microcrystalline cellulose + Guar Gum (Avicel CE-15) | 7.5 |
| 8 | Aspartame | 6 |
| 9 | Orange flavour Silesia | 3.75 |
| 10 | Magnesium stearate non bovine | 1.125 |
| | | 150 |

The ingredients 1-9, mirtazapine, Pearlitol, magnesium carbonate heavy, Avicel PH 102, L-HPC, crospovidone, Avicel CE-15, aspartame and Orange flavour are mixed, sieved and remixed. Then ingredient 10, magnesium stearate, is added to the blend, mixed and the blend is ready for compression. The tablets are compressed using direct compression.

The results were as follows:
Disintegration: 18 sec (37°C water)
Hardness: 18 - 26 N
Friability: 0.32%

### Example 6

### Compounding of orodispersible tablets containing mirtazapine using direct compression

**Table 7: Composition**

| | Ingredient | mg per tablet |
|---|---|---|
| 1 | Mirtazapine | 15 |
| 2 | Mannitol DC (Pearlitol SD 200) | 60 |
| 3 | Magnesium Carbonate Heavy | 7.5 |
| 4 | Microcrystalline Cellulose (Avicel PH-102) | 30.375 |
| 5 | Hydroxypropyl cellulose low substituted (L-HPC, LH-11) | 6 |
| 6 | L-Methionine | 0.75 |
| 7 | Crospovidone (Polyplasdone XL-10) | 12 |
| 8 | Microcrystalline cellulose + Guar Gum (Avicel CE-15) | 7.5 |
| 9 | Aspartame | 6 |
| 10 | Orange flavour Silesia | 3.75 |
| 11 | Magnesium stearate non bovine | 1.125 |
| | | 150 |

The ingredients 1 - 10, mirtazapine, Pearlitol, magnesium carbonate heavy, Avicel PH 102, L-HPC, L-Methionine, crospovidone, Avicel CE-15, aspartame and Orange flavour are mixed, sieved and remixed. Then ingredient 11, magnesium stearate, is added to the blend, mixed and the blend is ready for compression. The tablets are compressed using direct compression.

The results were as follows:
Disintegration: 19 sec (37°C water)
Hardness: 21 - 29 N
Friability: 0.27%

### Example 7

### Compounding of orodispersible tablets containing mirtazapine using wet granulation

**Table 8: Composition**

| | | |
|---|---|---|
| | Ingredient | mg per tablet |
| 1 | Mirtazapine | 45 |
| 2 | Mannitol DC (Pearlitol SO 200) | 33.75 |
| 3 | Microcrystalline cellulose (Avicel PH 102) | 91.125 |
| 4 | Magnesium Carbonate Heavy | 22.5 |
| 5 | Hydroxypropyl cellulose low substituted (L-HPC, LH-11) | 9 |
| 6 | Crospovidone (Polyplasdone XL-10) | 18 |
| 7 | Silica Colloidal anhydrous (Aerosil 200) | 4.5 |
| 8 | L-Methionine | 0.45 |
| 9 | Purified water | quantum satis |
| 10 | Mannitol DC (Pearlitol SD 200) | 140.175 |
| 11 | Hydroxypropyl cellulose low substituted (L-HPC, LH-11) | 9 |
| 12 | Crospovidone (Polyplasdone XL-10) | 18 |
| 13 | Microcrystalline cellulose + Guar Gum (Avicel | 22.5 |
| | CE-15) | |
| 14 | Aspartame | 18 |
| 15 | Orange flavour Silesia | 11.25 |
| 16 | Magnesium stearate non bovine | 6.75 |
| | | 450 |

The ingredients 1-7, Avicel PH 102, mirtazapine, Pearlitol SD 200, Aerosil 200, Polyplasdone XL-10, L-HPC and magnesium carbonate heavy are mixed. Then the ingredients are sieved and remixed. Ingredient 8, L-methionine, is dissolved in ingredient 9, purified water, and the blend is wetted with that solution, granulated, sieved, dried and sieved again. Ingredients 10 -15, Orange flavour Silesia, aspartame, Avicel CE-15, Polyplasdone XL-10, L-HPC and Pearlitol SD 200 are added in this order to the blend and mixed. Finally ingredient 16, magnesium stearate, Is added to the blend, mixed and the blend is ready for compression.

The results were as follows:
Disintegration: <25 sec (37°C water)
Hardness: 35 - 55 N
Friability: <1%

### Example 8

### Bio-study of the Mirtazapine Orodispersible tablets

The bio-study was a comparative, randomized, single-dose, 3-way crossover bio-availabllity study of (A) Actavis 30 mg Orodispersible Mirtazapine tablets (proportionally scaled up according to example 7), (B) Organon Laboratories Limited (Zispin®) 30 mg Mirtazapine tablets and (C) Organon Laboratories Limited (Remergil®Soltab^{™}) 30 mg Orodispersible Mirtazapine tablets, in healthy adult males under fasting conditions.
The results were as follows:

**Table 9: Mirtazapine in Plasma, ratios of LSM % (90% Confidence Intervals derived from the In transformed parameters)**

| Parameter | Actavis (A) vs. Organon ( Zispin^{®} ) (B) | Actavis (A) vs. Organon (Remergil^{®]} Soltab ^{™} (C) |
|---|---|---|
| AUC 0-t | 103.1% (99.4% - 107.0%) | 104.1% (100.3% - 108.0%) |
| AUCinf | 102.8% (99.1% - 106.7%) | 103.8% (100.1% - 107.6%) |
| Cmax | 109.8% (102.2% - 118.0%) | 115.1% (107.1%) - 123.7%) |

**Table 10: N-demethylmirtazapine in Plasma, ratios of LSM % (90% Confidence Intervals)**

| Parameter | Actavis (A) vs. Organon ( Zispin^{®}) (B) | Actavis (A) vs. Organon (Remergil^{®I} Soltab^{™} (C) |
|---|---|---|
| AUC 0-t | 99.9% (97.9% - 102.1%) | 99.4% (97.3% - 101.5%) |
| AUCinf | 99.6% (97.6% - 101.6%) | 99.4% (97.4% - 101.4%) |
| Cmax | 100.6% (95.6% - 105.8%) | 103.8% (98.7% - 109.3%) |

Based on the above results, it is concluded that the Actavis tablets (A) are bio-equivalent to the both the conventional tablets and the orodispersible tablets from Oragon (B and C, respectively).

### Example 9

### Compounding of orodispersible tablets containing olanzapine using wet granulation

Olanzapine is a psychotropic agent that belongs to the thiobenzodiazepine class. It is sensitive to both light and moisture, Insoluble in water and neutral pH but more soluble at acidic pH.
Because of the instability of olanzapine which is both light and moisture sensitive, trials were done where olanzapine was added extragranulary.

The particles size of olanzapine must be at less than 100 µm in size.

**Table 11: Composition**

| | Ingredient | mg per tablet |
|---|---|---|
| 1 | Microcrystalline cellulose (Avicel PH 102) | 21 |
| 2 | Hydroxypropyl cellulose low substituted (L-HPC, LH-11) | 2.1 |
| 3 | Silica Colloidal anhydrous (Aerosil 200) | 1.05 |
| 4 | Crospovidone (Polyplasdone XL-10) | 4.9 |
| 5 | Magnesium Carbonate Heavy | 21.53 |
| 6 | Purified water | quantum satis |
| 7 | Olanzapine, | 5.0 |
| 8 | Hydroxypropyl cellulose low substituted (L-HPC, LH-11) | 1.4 |
| 9 | Crospovidone (Polyplasdone XL-10) | 2.1 |
| 10 | Microcrystalline cellulose (Avicel PH 102) | 3.5 |
| 11 | Microcrystalline cellulose + Guar Gum (Avicel CE-15) | 3.5 |
| 12 | Silica Colloidal anhydrous (Aerosil 200) | 0.175 |
| 13 | Peppermint Flavour natural Silesia | 0.42 |
| 14 | Aspartame | 2.8 |
| 15 | Magnesium stearate non bovine | 0.525 |
| | | 70.0 |

The ingredients 1-5, Avicel PH 102, L-HPC, Aerosil 200, Polyplasdone XL-10 and magnesium carbonate heavy are added to a bowl In this order and mixed. Then the ingredients are sieved and remixed. The blend is wetted with ingredient 6, granulated, sieved, dried and sieved again. Ingredients 7 - 14, olanzapine, L-HPC, Polyplasdone XL-10, Avicel PH 102, Avicel CE-15, Aerosil 200, Peppermint flavour natural and aspartame are added to a bowl and mixed. Ingredients 7-14 are then added to the dry granules and mixed. Finally ingredient 15, magnesium stearate, is added to the blend, mixed and the blend is ready for compression.
The results were as follows:
Disintegration: 9 sec (37°C water)
Oral disintegration time: <15 sec
Hardness: 38 - 60 N
Friability: 0.0%

### Example 10

### Compounding of orodispersible tablets containing risperidone using direct compression

Risperidone is an antipsychotic agent that belongs to the benzisoxazole-derivatives.

**Table 12: Composition**

| | Ingredient | mg per tablet |
|---|---|---|
| 1 | Risperidone | 1 |
| 2 | Mannitol DC (Pearlitol SD 200) | 20.98 |
| 3 | Magnesium Carbonate Heavy | 17.5 |
| 4 | Microcrystalline cellulose (Avicel PH 102) | 14 |
| 5 | Hydroxypropyl cellulose low substituted (L-HPC, LH-11) | 2.8 |
| 6 | Crospovidone (Polyplasdone XL-10) | 5.6 |
| 7 | Microcrystalline cellulose + Guar Gum (Avicel CE-15) | 3.5 |
| 8 | Aspartame | 2.8 |
| 9 | Peppermint Flavour | 0.42 |
| 10 | Silica Colloidal anhydrous (Aerosil 200) | 0.7 |
| 11 | Magnesium stearate non bovine | 0.7 |
| | | 70.0 |

The ingredients 1 - 10, risperidone, Pearlitol SD 200, magnesium carbonate heavy, Avicel PH 102, L-HPC, Polyplasdone XL-10, Avicel CE-15, aspartame, Peppermint flavour and Aerosil 200 are added to a bin, mixed, sieved and then re-mixed. Then ingredient 11, magnesium stearate, is added to the blend, mixed and the blend is ready for compression.

The results were as follows:
Disintegration: 8 sec
Hardness: 10 - 17 N
Friability: 0.24%

## Claims

1. A dispersible dosage form for administrating a medicament, which disintegrates in less than 3 minutes when in contact with water, comprising magnesium carbonate heavy as a dispersant and an active pharmaceutical substance.

2. The dosage form of claim 1 comprising magnesium carbonate heavy in the range of 1% to 85% by weight.

3. The dosage form of claim 1 comprising magnesium carbonate heavy In the range of 4 to 65% by weight.

4. The dosage form of claim 1, which is a dispersible tablet intended for dispersion in water prior to administration.

5. The dosage form of claim 4, wherein the dosage form disintegrates In less than 3 minutes at 15°C in water.

6. The dosage form of claim 5, wherein the dosage form disintegrates In less than 2 minutes at 15°C in water.

7. The dosage form of claim 6, wherein the dosage form disintegrates in less than 1 minute at 15°C in water.

8. The dosage form of claim 1, which Is an orodispersible tablet.

9. The dosage form of claim 8, which disintegrates In less than 1 minute at 37°C in water.

10. The dosage form of claim 8, which disintegrates in less than 30 seconds at 37°C in water.

11. The dosage form of any of the aforementioned claims, comprising as active substance mirtazepine or a pharmaceutically acceptable salt thereof.

12. The dosage form of any of claims 1-10 comprising as active substance olanzapine or a pharmaceutically acceptable salt thereof.

13. The dosage form of any of claims 1-10 comprising as an active substance risperidone or a pharmaceutically acceptable salt thereof.

14. The dosage form of any of claims 1-10 comprising as an active substance lamotrigine or a pharmaceutically acceptable salt thereof.

15. The dosage form of any of the aforementioned claims, wherein the dosage form is compressed to a tablet.

16. The dosage form of claim 15, wherein said tablet is a direct compression tablet.

17. The dosage form of claim 15, wherein the compounding of said tablet is made using wet granulation.

18. The dosage form of any of claims 15-17 compressed to a tablet having hardness in the range of 15 to 170 N.

19. The dosage form of any of the aforementioned claims, wherein the dosage form is a dispersible and/or orodispersible tablet, wherein the friability of the tablet is less than 1%.

20. The dosage form of any of the aforementioned claims, comprising microcrystalline cellulose and guar gum.

21. The dosage form of claim 20 comprising microcrystalline cellulose and guar gum of the type Avicel CE-15.

22. A dispersible pharmaceutical dosage form comprising lamotrigine as an active ingredient, and further comprising:
- In the range of about 50-65 wt% magnesium carbonate heavy;
- In the range of about 10-30 wt% fine particle microcrystalline cellulose;
- In the range of about 3-10 wt% low-substituted hydroxypropyl cellulose;
- In the range of about 5-15 wt% crospovldone;
- in the range of about 4-20 wt% Avicel CE-15 mixture of microcrystalline cellulose and guar gum

23. The dosage form of claim 22 comprising:
- about 2,5 wt% lamotrigine
- about 61 wt% magnesium carbonate heavy;
- about 15 wt% fine particle microcrystalline cellulose;
- about 4 wt% low-substituted hydroxypropyl cellulose;
- about 8 wt% crospovidone ; and
- about 5 wt% Avicel® CE-15 mixture of microcrystalline cellulose and guar gum.

24. A dispersible pharmaceutical dosage form comprising mirtazepine as an active ingredient, and further comprising:
- in the range of about 30-65 wt% magnesium carbonate heavy;
- in the range of about 10-30 wt% fine particle microcrystalline cellulose;
- in the range of about 3-10 wt% low-substituted hydroxypropyl cellulose;
- in the range of about 5-15 wt% crospovidone;
- in the range of about 4-20 wt% Avicel® CE-15 mixture of microcrystalline cellulose and guar gum.

25. The dosage form of claim 24 comprising:
- about 10 wt% mirtazepine
- about 5 wt% magnesium carbonate heavy;
- about 20 wt% fine particle microcrystalline cellulose;
- about 4 wt% low-substituted hydroxypropyl cellulose;
- about 8 wt% crospovidone; and
- about 5 wt% Avicel® CE-15 mixture of microcrystalline cellulose and guar gum

26. The dosage form of claim 24 comprising:
- about 10 wt% mirtazepine
- about 42 wt% magnesium carbonate heavy;
- about 15 wt% fine particle microcrystalline cellulose;
- about 4 wt% low-substituted hydroxypropyl cellulose;
- about 8 wt% crospovidone; and
- about 5 wt% Avicel® CE-15 mixture of microcrystalline cellulose and guar gum.

27. A dispersible pharmaceutical dosage form comprising olanzapine as an active ingredient, and further comprising:
- in the range of about 20-60 wt% magnesium carbonate heavy;
- in the range of about 15-35 wt% fine particle microcrystalline cellulose;
- in the range of about 3-10 wt% low-substituted hydroxypropyl cellulose;
- in the range of about 5-15 wt% crospovidone;
- in the range of about 20-45 wt% Avicel® CE-15 mixture of microcrystalline cellulose and guar gum.

28. The dosage form of claim 27 comprising:
- about 7 wt% olanzapine;
- about 31 wt% magnesium carbonate heavy;
- about 30 wt% fine particle microcrystalline cellulose;
- about 5 wt% low-substituted hydroxypropyl cellulose;
- about 10 wt% crospovidone; and
- about 5 wt% Avicel® CE-15 mixture of microcrystalline cellulose and guar gum.

29. A dispersible pharmaceutical dosage form comprising risperidone as an active ingredient, and further comprising:
- in the range of about 20-60 wt% magnesium carbonate heavy;
- in the range of about 15-35 wt% fine particle microcrystalline cellulose;
- in the range of about 3-10 wt% low-substituted hydroxypropyl cellulose;
- in the range of about 5-15 wt% crospovidone;
- in the range of about 4-20 wt% Avicel® CE-15 mixture of microcrystalline cellulose and guar gum.

30. The dosage form of claim 29 comprising:
- about 1,4 wt% olanzapine;
- about 25 wt% magnesium carbonate heavy;
- about 20 wt% fine particle microcrystalline cellulose;
- about 4 wt% low-substituted hydroxypropyl cellulose;
- about 8 wt% crospovidone; and
- about 5 wt% Avicel® CE-15 mixture of microcrystalline cellulose and guar gum.

## Patentansprüche

1. Dispergierbare Dosierungsform zum Verabreichen eines Medikaments, die sich in weniger als 3 Minuten bei Kontakt mit Wasser zersetzt, umfassend schweres Magnesiumcarbonat als Dispergiermittel und einen pharmazeutischen Wirkstoff.

2. Dosierungsform nach Anspruch 1, umfassend schweres Magnesiumcarbonat im Bereich von 1 bis 85 Gewichtsprozent.

3. Dosierungsform nach Anspruch 1, umfassend schweres Magnesiumcarbonat im Bereich von 4 bis 65 Gewichtsprozent.

4. Dosierungsform nach Anspruch 1, die eine dispergierbare Tablette ist, die zur Dispersion in Wasser vor der Verabreichung bestimmt ist.

5. Dosierungsform nach Anspruch 4, wobei sich die Dosierungsform in weniger als 3 Minuten bei 15°C in Wasser zersetzt.

6. Dosierungsform nach Anspruch 5, wobei sich die Dosierungsform in weniger als 2 Minuten bei 15°C in Wasser zersetzt.

7. Dosierungsform nach Anspruch 6, wobei sich die Dosierungsform in weniger als 1 Minute bei 15°C in Wasser zersetzt.

8. Dosierungsform nach Anspruch 1, die eine oral dispergierbare Tablette ist.

9. Dosierungsform nach Anspruch 8, die sich in weniger als 1 Minute bei 37°C in Wasser zersetzt.

10. Dosierungsform nach Anspruch 8, die sich in weniger als 30 Sekunden bei 37°C in Wasser zersetzt.

11. Dosierungsform nach einem der vorangehenden Ansprüche, umfassend Mirtazepin oder ein pharmazeutisch annehmbares Salz davon als Wirkstoff.

12. Dosierungsform nach einem der Ansprüche 1 bis 10, umfassend Olanzapin oder ein pharmazeutisch annehmbares Salz davon als Wirkstoff.

13. Dosierungsform nach einem der Ansprüche 1 bis 10, umfassend Risperidon oder ein pharmazeutisch annehmbares Salz davon als Wirkstoff.

14. Dosierungsform nach einem der Ansprüche 1 bis 10, umfassend Lamotrigin oder ein pharmazeutisch annehmbares Salz davon als Wirkstoff.

15. Dosierungsform nach einem der vorangehenden Ansprüche, wobei die Dosierungsform zu einer Tablette verpresst ist.

16. Dosierungsform nach Anspruch 15, wobei die Tablette eine direkt verpresste Tablette ist.

17. Dosierungsform nach Anspruch 15, wobei die Mischungsherstellung der Tablette unter Anwendung einer Nassgranulierung erfolgt.

18. Dosierungsform nach einem der Ansprüche 15 bis 17, die zu einer Tablette mit einer Härte im Bereich von 15 bis 170 N verpresst ist.

19. Dosierungsform nach einem der vorangehenden Ansprüche, wobei die Dosierungsform eine dispergierbare und/oder oral dispergierbare Tablette ist, wobei die Bröckligkeit der Tablette geringer als 1% ist.

20. Dosierungsform nach einem der vorangehenden Ansprüche, umfassend mikrokristalline Zellulose und Guargummi.

21. Dosierungsform nach Anspruch 20, umfassend mikrokristalline Zellulose und Guargummi vom Typ Avicel CE-15.

22. Dispergierbare pharmazeutische Dosierungsform, umfassend Lamotrigin als Wirkstoff und des Weiteren umfassend:
- schweres Magnesiumcarbonat im Bereich von etwa 50 bis 65 Gewichtsprozent;
- mikrokristalline Feinpartikel-Zellulose im Bereich von etwa 10 bis 30 Gewichtsprozent;
- niedrig substituierte Hydroxypropylzellulose im Bereich von etwa 3 bis 10 Gewichtsprozent;
- Crospovidon im Bereich von etwa 5 bis 15 Gewichtsprozent;
- Avicel CE-15 Mischung aus mikrokristalliner Zellulose und Guargummi im Bereich von etwa 4 bis 20 Gewichtsprozent.

23. Dosierungsform nach Anspruch 22, umfassend:
- etwa 2,5 Gewichtsprozent Lamotrigin;
- etwa 61 Gewichtsprozent schweres Magnesiumcarbonat;
- etwa 15 Gewichtsprozent mikrokristalline Feinpartikel-Zellulose;
- etwa 4 Gewichtsprozent niedrig substituierte Hydroxypropylzellulose;
- etwa 8 Gewichtsprozent Crospovidon; und
- etwa 5 Gewichtsprozent Avicel® CE-15 Mischung aus mikrokristalliner Zellulose und Guargummi.

24. Dispergierbare pharmazeutische Dosierungsform, umfassend Mirtazepin als Wirkstoff und des Weiteren umfassend:
- schweres Magnesiumcarbonat im Bereich von etwa 30 bis 65 Gewichtsprozent;
- mikrokristalline Feinpartikel-Zellulose im Bereich von etwa 10 bis 30 Gewichtsprozent;
- niedrig substituierte Hydroxypropylzellulose im Bereich von etwa 3 bis 10 Gewichtsprozent;
- Crospovidon im Bereich von etwa 5 bis 15 Gewichtsprozent;
- Avicel® CE-15 Mischung aus mikrokristalliner Zellulose und Guargummi im Bereich von etwa 4 bis 20 Gewichtsprozent.

25. Dosierungsform nach Anspruch 24, umfassend:
- etwa 10 Gewichtsprozent Mirtazepin;
- etwa 5 Gewichtsprozent schweres Magnesiumcarbonat;
- etwa 20 Gewichtsprozent mikrokristalline Feinpartikel-Zellulose;
- etwa 4 Gewichtsprozent niedrig substituierte Hydroxypropylzellulose;
- etwa 8 Gewichtsprozent Crospovidon; und
- etwa 5 Gewichtsprozent Avicel® CE-15 Mischung aus mikrokristalliner Zellulose und Guargummi.

26. Dosierungsform nach Anspruch 24, umfassend:
- etwa 10 Gewichtsprozent Mirtazepin;
- etwa 42 Gewichtsprozent schweres Magnesiumcarbonat;
- etwa 15 Gewichtsprozent mikrokristalline Feinpartikel-Zellulose;
- etwa 4 Gewichtsprozent niedrig substituierte Hydroxypropylzellulose;
- etwa 8 Gewichtsprozent Crospovidon; und
- etwa 5 Gewichtsprozent Avicel® CE-15 Mischung aus mikrokristalliner Zellulose und Guargummi.

27. Dispergierbare pharmazeutische Dosierungsform, umfassend Olanzapin als Wirkstoff und des Weiteren umfassend:
- schweres Magnesiumcarbonat im Bereich von etwa 20 bis 60 Gewichtsprozent;
- mikrokristalline Feinpartikel-Zellulose im Bereich von etwa 15 bis 35 Gewichtsprozent;
- niedrig substituierte Hydroxypropylzellulose im Bereich von etwa 3 bis 10 Gewichtsprozent;
- Crospovidon im Bereich von etwa 5 bis 15 Gewichtsprozent;
- Avicel® CE-15 Mischung aus mikrokristalliner Zellulose und Guargummi im Bereich von etwa 20 bis 45 Gewichtsprozent.

28. Dosierungsform nach Anspruch 27, umfassend:
- etwa 7 Gewichtsprozent Olanzapin;
- etwa 31 Gewichtsprozent schweres Magnesiumcarbonat;
- etwa 30 Gewichtsprozent mikrokristalline Feinpartikel-Zellulose;
- etwa 5 Gewichtsprozent niedrig substituierte Hydroxypropylzellulose;
- etwa 10 Gewichtsprozent Crospovidon; und
- etwa 5 Gewichtsprozent Avicel® CE-15 Mischung aus mikrokristalliner Zellulose und Guargummi.

29. Dispergierbare pharmazeutische Dosierungsform, umfassend Risperidon als Wirkstoff und des Weiteren umfassend:
- schweres Magnesiumcarbonat im Bereich von etwa 20 bis 60 Gewichtsprozent;
- mikrokristalline Feinpartikel-Zellulose im Bereich von etwa 15 bis 35 Gewichtsprozent;
- niedrig substituierte Hydroxypropylzellulose im Bereich von etwa 3 bis 10 Gewichtsprozent;
- Crospovidon im Bereich von etwa 5 bis 15 Gewichtsprozent;
- Avicel® CE-15 Mischung aus mikrokristalliner Zellulose und Guargummi im Bereich von etwa 4 bis 20 Gewichtsprozent.

30. Dosierungsform nach Anspruch 29, umfassend:
- etwa 1,4 Gewichtsprozent Risperidon;
- etwa 25 Gewichtsprozent schweres Magnesiumcarbonat
- etwa 20 Gewichtsprozent mikrokristalline Feinpartikel-Zellulose;
- etwa 4 Gewichtsprozent niedrig substituierte Hydroxypropylzellulose;
- etwa 8 Gewichtsprozent Crospovidon; und
- etwa 5 Gewichtsprozent Avicel® CE-15 Mischung aus mikrokristalliner Zellulose und Guargummi.

## Revendications

1. Forme posologique dispersible pour l'administration d'un médicament, qui se désagrège en moins de 3 minutes au contact de l'eau, comprenant du carbonate de magnésium lourd en tant que dispersant et un principe actif pharmaceutique.

2. Forme posologique selon la revendication 1, qui comprend du carbonate de magnésium lourd dans la plage de 1 % à 85 % en poids.

3. Forme posologique selon la revendication 1, qui comprend du carbonate de magnésium lourd dans la plage de 4 à 65 en poids.

4. Forme posologique selon la revendication 1, qui est un comprimé dispersible destiné à être dispersé dans l'eau avant l'administration.

5. Forme posologique selon la revendication 4, la forme posologique se désagrégeant en moins de 3 minutes à 15°C dans l'eau.

6. Forme posologique selon la revendication 5, la forme posologique se désagrégeant en moins de 2 minutes à 15°C dans l'eau.

7. Forme posologique selon la revendication 6, la forme posologique se désagrégeant en moins de 1 minute à 15°C dans l'eau.

8. Forme posologique selon la revendication 1, qui est un comprimé orodispersible.

9. Forme posologique selon la revendication 8, qui se désagrège en moins de 1 minute à 37°C dans l'eau.

10. Forme posologique selon la revendication 8, qui se désagrège en moins de 30 secondes à 37°C dans l'eau.

11. Forme posologique selon l'une quelconque des revendications précédentes, qui comprend en tant que principe actif de la mirtazépine ou un sel pharmaceutiquement acceptable de celle-ci.

12. Forme posologique selon l'une quelconque des revendications 1 à 10, qui comprend en tant que principe actif de l'olanzapine ou un sel pharmaceutiquement acceptable de celle-ci.

13. Forme posologique selon l'une quelconque des revendications 1 à 10, qui comprend en tant que principe actif de la rispéridone ou un sel pharmaceutiquement acceptable de celle-ci.

14. Forme posologique selon l'une quelconque des revendications 1 à 10, qui comprend en tant que principe actif de la lamotrigine ou un sel pharmaceutiquement acceptable de celle-ci.

15. Forme posologique selon l'une quelconque des revendications précédentes, dans laquelle la forme posologique est compressée en un comprimé.

16. Forme posologique selon la revendication 15, dans laquelle ledit comprimé est un comprimé obtenu par compression directe.

17. Forme posologique selon la revendication 15, dans laquelle la préparation dudit comprimé est réalisée en utilisant la granulation par voie humide.

18. Forme posologique selon l'une quelconque des revendications 15 à 17, compressée en un comprimé ayant une dureté dans la plage de 15 à 170 N.

19. Forme posologique selon l'une quelconque des revendications précédentes, la forme posologique étant un comprimé dispersible et/ou orodispersible, la friabilité du comprimé étant inférieure à 1 %.

20. Forme posologique selon l'une quelconque des revendications précédentes, comprenant de la cellulose microcristalline et de la gomme de guar.

21. Forme posologique selon la revendication 20, comprenant de la cellulose microcristalline et de la gomme de guar du type Avicel® CE-15.

22. Forme posologique pharmaceutique dispersible comprenant de la lamotrigine en tant que principe actif, et comprenant en outre :
- d'environ 50 à 65 % en poids de carbonate de magnésium lourd ;
- d'environ 10 à 30 % en poids de fines particules de cellulose microcristalline ;
- d'environ 3 à 10 % en poids d'hydroxypropylcellulose faiblement substituée ;
- d'environ 5 à 15 % en poids de crospovidone ;
- d'environ 4 à 20 % en poids de mélange Avicel® CE-15 constitué de cellulose microcristalline et de gomme de guar.

23. Forme posologique selon la revendication 22, qui comprend :
- environ 2,5 % en poids de lamotrigine ;
- environ 61 % en poids de carbonate de magnésium lourd ;
- environ 15 % en poids de fines particules de cellulose microcristalline ;
- environ 4 % en poids d'hydroxypropylcellulose faiblement substituée ;
- environ 8 % en poids de crospovidone ; et
- environ 5 % en poids de mélange Avicel® CE-15 constitué de cellulose microcristalline et de gomme de guar.

24. Forme posologique pharmaceutique dispersible comprenant de la mirtazépine en tant que principe actif, et comprenant en outre :
- d'environ 30 à 65 % en poids de carbonate de magnésium lourd ;
- d'environ 10 à 30 % en poids de fines particules de cellulose microcristalline ;
- d'environ 3 à 10 % en poids d'hydroxypropylcellulose faiblement substituée ;
- d'environ 5 à 15 % en poids de crospovidone ;
- d'environ 4 à 20 % en poids de mélange Avicel® CE-15 constitué de cellulose microcristalline et de gomme de guar.

25. Forme posologique selon la revendication 24, qui comprend :
- environ 10 % en poids de mirtazépine ;
- environ 5 % en poids de carbonate de magnésium lourd ;
- environ 20 % en poids de fines particules de cellulose microcristalline ;
- environ 4 % en poids d'hydroxypropylcellulose faiblement substituée ;
- environ 8 % en poids de crospovidone ; et
- environ 5 % en poids de mélange Avicel® CE-15 constitué de cellulose microcristalline et de gomme de guar.

26. Forme posologique selon la revendication 24, qui comprend :
- environ 10 % en poids de mirtazépine ;
- environ 42 % en poids de carbonate de magnésium lourd ;
- environ 15 % en poids de fines particules de cellulose microcristalline ;
- environ 4 % en poids d'hydroxypropylcellulose faiblement substituée ;
- environ 8 % en poids de crospovidone ; et
- environ 5 % en poids de mélange Avicel® CE-15 constitué de cellulose microcristalline et de gomme de guar.

27. Forme posologique pharmaceutique dispersible comprenant de l'olanzapine en tant que principe actif, et comprenant en outre :
- d'environ 20 à 60 % en poids de carbonate de magnésium lourd ;
- d'environ 15 à 35 % en poids de fines particules de cellulose microcristalline ;
- d'environ 3 à 10 % en poids d'hydroxypropylcellulose faiblement substituée ;
- d'environ 5 à 15 % en poids de crospovidone ;
- d'environ 20 à 45 % en poids de mélange Avicel® CE-15 constitué de cellulose microcristalline et de gomme de guar.

28. Forme posologique selon la revendication 27, qui comprend :
- environ 7 % en poids d'olanzapine ;
- environ 31 % en poids de carbonate de magnésium lourd ;
- environ 30 % en poids de fines particules de cellulose microcristalline ;
- environ 5 % en poids d'hydroxypropylcellulose faiblement substituée ;
- environ 10 % en poids de crospovidone ; et
- environ 5 % en poids de mélange Avicel® CE-15 constitué de cellulose microcristalline et de gomme de guar.

29. Forme posologique pharmaceutique dispersible comprenant de la rispéridone en tant que principe actif, et comprenant en outre :
- d'environ 20 à 60 % en poids de carbonate de magnésium lourd ;
- d'environ 15 à 35 % en poids de fines particules de cellulose microcristalline ;
- d'environ 3 à 10 % en poids d'hydroxypropylcellulose faiblement substituée ;
- d'environ 5 à 15 % en poids de crospovidone ;
- d'environ 4 à 20 % en poids de mélange Avicel® CE15 constitué de cellulose microcristalline et de gomme de guar.

30. Forme posologique selon la revendication 29, qui comprend :
- environ 1,4 % en poids de rispéridone;
- environ 25 % en poids de carbonate de magnésium lourd ;
- environ 20 % en poids de fines particules de cellulose microcristalline ;
- environ 4 % en poids d'hydroxypropylcellulose faiblement substituée ;
- environ 8 % en poids de crospovidone ; et
- environ 5 % en poids de mélange Avicel® CE-15 constitué de cellulose microcristalline et de gomme de guar.
